# EUROPÄISCHE PATENTSCHRIFT

(11) **EP 2 760 989 B1**
(45) Veröffentlichungstag und Bekanntmachung des Hinweises auf die Patenterteilung: **28.10.2020**
(21) Anmeldenummer: 11776343.3
(22) Anmeldetag: 29.09.2011
(51) Int. Cl.: C12M 1/107

(54) **BIOGASANLAGE UND VERFAHREN ZUM BETREIBEN EINER BIOGASANLAGE**
BIOGAS PLANT AND METHOD FOR OPERATING A BIOGAS PLANT
INSTALLATION DE BIOGAZ ET PROCÉDÉ POUR FAIRE FONCTIONNER UNE INSTALLATION DE BIOGAZ

(43) Veröffentlichungstag der Anmeldung: 06.08.2014
(73) Patentinhaber: Kompoferm GmbH, 33428 Marienfeld (DE)
(72) Erfinder: EGGERSMANN, Karlgünter, 33428 Marienfeld (DE)
(74) Vertreter: Schober, Mirko
(86) Internationale Anmeldenummer: PCT/EP2011/004853
(87) Internationale Veröffentlichungsnummer: WO 2013/044935

(56) Entgegenhaltungen:
- EP-A1- 1 428 868
- EP-A2- 0 350 455
- EP-A2- 1 191 086
- WO-A1-2010/054827
- WO-A2-2005/054423
- WO-A2-2008/095685
- WO-A2-2010/063709
- DE-A1- 19 947 339
- DE-A1-102007 058 548
- DE-C1- 3 134 980
- US-A- 5 269 634

## Beschreibung

### TECHNISCHES GEBIET

Die Erfindung betrifft eine Biogasanlage mit mindestens einem Fermenter zur Erzeugung von Biogas sowie ein Verfahren zum Betrieb einer Biogasanlage.

### STAND DER TECHNIK

Derartige Biogasanlagen dienen der Erzeugung methanhaltigen Biogases, welches zur Energiegewinnung, beispielsweise mittels eines Blockheizkraftwerks verwendet wird. Dabei arbeiten die Fermenter im Bachbetrieb, d.h. zwischen Befüllen und Entladen der Fermenter mit der zu vergärenden bzw. vergorenen Biomasse findet kein stationärer Betrieb eines solchen Fermenters statt. Vielmehr kommt es zu Beginn und zum Ende der Biomassevergärung zu An- und Abfahrphasen, in denen Gase bzw. Gas-Luft-Gemische produziert werden, die insbesondere aufgrund eines zu niedrigen Methangehalts keiner sinnvollen Verwertung zugeführt werden können. Derzeit werden diese Gasgemische entweder abgefackelt oder einer anderen Art der Abluftbehandlung zugeführt. Durch die hierbei entstehenden Verluste potenziell verwertbaren Methans sinkt der Wirkungsgrad einer solchen Anlage. Weitere Wirkungsgrad mindernde Verluste bei konventionellen Anlagen entstehen dadurch, dass die Temperatur im Fermenter höher ist als die Temperatur, mit der das Biogas konventionellen Verwertungseinrichtungen zugeführt werden muss. Dadurch kommt es zum einen zu Wärmeverlusten des Fermenters, zum anderen zur notwendigen Aufwendung von Kühlleistung für das Biogas.

Aus WO 2010/06709 A2 ist ein Verfahren zur Verminderung des Methanschlupfes beim Anfahren und Abschalten von Biogasfermentern bekannt. Das aus einem oder mehreren Fermentern erzeugte, methanhaltige Biogas wird mittels einer Biogasaufbereitung aufbereitet. Dabei wird das methanhaltige Biogas dadurch verwertbar gemacht, dass die Biogasaufbereitung das Biogas hinsichtlich seiner Methankonzentration aufkonzentriert, indem andere Bestandteile über eine Leitung ausgeschleust werden. Über eine Mess- und Steuereinrichtung kann dann entschieden werden, ob die danach vorliegende Konzentration an Methan hoch genug ist, um das Biogas einer Verwertung zuzuführen. Falls nicht, kann das Biogas beispielsweise wieder in einen oder eine Mehrzahl Fermenter zurückgeleitet werden. Es werden lediglich Mengenströme aufgeteilt. Das beschriebene Verfahren ist jedoch nicht in der Lage, dauerhaft ein verwertbares Gasgemisch einer Verwertung zuzuführen, ohne steuerungstechnisch auf das Anfahren und Abfahren der Fermenter einzuwirken und eine teure Gasaufbereitung einzusetzen.

Aus EP 1 428 868 A1 ist eine Vorrichtung zur anaeroben Fermentation von Biomasse bekannt. Diese verfügt über einen Zwischenspeicher zur Speicherung von Biogas, welches einen gewissen Druck überschreitet. Dazu werden Ventile geöffnet oder geschlossen. Diese ermöglichen jedoch keine Regelung des Mengendurchflusses.

### DIE ERFINDUNG

Der Erfindung liegt nun die Aufgabe zugrunde, eine Biogasanlage und ein Verfahren zum Betreiben einer Biogasanlage aufzuzeigen, welche die geschilderten Nachteile vermeiden oder zumindest reduzieren und so einen höheren Wirkungsgrad der Biogasanlage ermöglichen.

Gelöst wird die Aufgabe durch eine Biogasanlage mit den Merkmalen des Anspruchs 1 und ein Verfahren zum Betrieb einer Biogasanlage mit den Merkmalen des Anspruchs 7. Die Merkmale der abhängigen Ansprüche betreffen vorteilhafte Ausführungsformen, welche einen positiven Beitrag zur Lösung der erfindungsgemäßen Aufgabe leisten.

Erfindungsgemäß ist vorgesehen, dass in einem Fermenter gewonnenes Biogas, welches einen Methangehalt unterhalb eines ersten vorgebbaren Grenzwertes aufweist, in einem ersten Gasspeicher zwischengespeichert wird. Dabei handelt es sich um jenes Biogas, welches in An- und Abfahrphasen entsteht und in konventionellen Anlagen in der Regel entsorgt werden muss. Dieses Biogas mit dem Methangehalt unterhalb eines ersten vorgebbaren Grenzwertes wird im Folgenden auch als "Schwachgas" bezeichnet. Vorteilhafterweise wird dieses Schwachgas mit Biogas, welches einen Methangehalt oberhalb eines zweiten vorgebbaren Grenzwertes aufweist, im Folgenden "Gutgas", gemischt und eine Biogasverwertungseinrichtung mit einer Mischung aus Gutgas und Schwachgas, im Folgenden als "Mischgas" bezeichnet, betrieben.

Um die Realisierung des Verfahrens zu ermöglichen, weist die erfindungsgemäße Biogasanlage mindestens zwei Gasspeicher auf, so dass ein erster Gasspeicher zur Speicherung von Schwachgas und ein zweiter Gasspeicher zur Speicherung von Gutgas zur Verfügung steht. Die Gasspeicher sind über Biogasleitungen mit Gasauslässen des mindestens einen Fermenters verbindbar und die Biogasverwertungseinrichtung ist derart mit den Biogasspeichern und/oder den Biogasleitungen verbindbar, dass es möglich ist, zum einen Biogas aus dem ersten Gasspeicher oder den diesen versorgenden Leitungen und zum anderen Biogas aus dem zweiten Gasspeicher oder den diesen versorgenden Leitungen zu entnehmen und die beiden Gase gleichzeitig der Biogasverwertungseinrichtung zuzuführen. Erfindungsgemäß weist dabei die Biogasanlage eine Steuereinrichtung zur Steuerung der Mengenverhältnisse der aus den Biogasleitungen und/oder den Biogasspeichern zur Biogasverwertungseinrichtung geführten Biogasströme auf. Durch diese Verschaltung ist es möglich, der Biogasverwertungseinrichtung verwertbare Gasgemische zuzuführen, auch wenn in An- oder Abfahrphasen des oder eines der Fermenter(s) temporär Gas erzeugt wird, dessen Methangehalt für eine Verwertung normalerweise zu niedrig wäre. Durch die Gasspeicher ist es dabei möglich, Gutgas oder Schwachgas zwischen zu speichern, so dass gemittelt über die Betriebszeit der Biogasanlage immer verwertbares Biogas als Mischgas erzeugt werden kann, auch von temporär ein zu hoher Schwachgasanteil erzeugt wird, der dann im Schwachgasspeicher zwischengespeichert wird. Das zwischengespeicherte Schwachgas kann dann in Betriebsphasen mit hohem Gutgasanteil dem Speicher wieder entnommen und dem Mischgas zugeführt werden. Im umgekehrten Fall ist es ebenso möglich, in Phasen mit hohem Gutgasanteil dieses zwischen zu speichern und in Phasen mit zu niedrigem Gutgasanteil dem Speicher wieder zu entnehmen, um ein verwertbares Mischgas zu erhalten.

Vorteilhafterweise werden dabei die Mengenverhältnisse von Gut- und Schwachgas so geregelt, dass sich der Methangehalt des Mischgases oberhalb eines dritten vorgebbaren Grenzwertes bewegt. Vorzugsweise ist die Regelung dabei derart realisiert, dass sie abhängig vom Methangehalt von Gut- und/ oder Schwachgas erfolgt, so dass bei schwankenden Methangehalten im Gut- und im Schwachgas dennoch sichergestellt ist, dass das Mischgas in seinem Methangehalt den dritten vorgebbaren Grenzwert überschreitet.

Erfindungsgemäß weist daher die Biogasanlage eine Messeinrichtung zur Messung des Methangehalts auf, die mit den Biogasleitungen und/oder den Biogasspeichern verbindbar ist, so dass vorteilhafterweise Werte über die Methangehalte sowohl der aktuell in den Fermentern produzierten und in den Biogasspeichern gespeicherten Gase und/oder des der Biogasverwertungseinrichtung zugeführten Mischgases zur Verfügung stehen.

Vorteilhafterweise wird der erste Grenzwert so gewählt, dass der Methangehalt im ersten Biogasspeicher, also der Methangehalt des Schwachgases, unterhalb der vom Sauerstoffgehalt abhängigen unteren Explosionsgrenze liegt. Dadurch werden die Investitionsgrenzen durch die geringeren Anforderungen an den Explosionsschutz der Anlage gesenkt.

Vorteilhafterweise liegt der zweite Grenzwert, also der untere Grenzwert für den Methangehalt des Gutgases, bei 37%, vorzugsweise bei 52%. Weiterhin ist es vorteilhaft, wenn der dritte Grenzwert, also der für den Mindestmethangehalt des Mischgases, bei 35%, vorzugsweise bei 50% liegt. Methangehalte oberhalb dieser Grenzwerte eröffnen Einsatzmöglichkeiten für gängige Typen von Blockheizkraftwerken und ermöglichen einen weitgehend unterbrechungsfreien Betrieb der Anlage.

Vorteilhafterweise kann zumindest phasenweise oder zyklisch Biogas oder Schwachgas aus einem Fermenter in diesen zurück geführt und so im Kreislauf gefahren werden. Der Fermenter weist hierzu einen Spülgaseinlauf, vorzugsweise im Bereich des Bodens des Fermenters, auf, der mit der entsprechenden Biogasleitung verbindbar ist und es so ermöglicht, die Biomasse zu deren Auflockerung mit Gutgas und/oder Schwachgas zu durchspülen und so eine bessere Drainage in der Biomasse zu gewährleisten.

Vorzugsweise ist der Spülgaseinlass ebenfalls mit einer Frischluftzuleitung verbindbar, was eine Frischluftspülung des Fermenters, insbesondere während des An- oder Abfahrbetriebs ermöglicht. Es ist dabei weiterhin vorteilhaft, wenn der Spülgaseinlass auch mit dem Schwachgas- und/oder dem Gutgasspeicher verbindbar ist, um die entsprechenden Spülbehandlungen auch mit Gas aus einem Reservoir durchführen zu können, beispielsweise wenn das Gas aufgrund der Anforderungen an die der Biogasverwertung zugeführte Gasmischung nicht direkt aus einem Fermenter entnommen werden kann, ohne den Betrieb der Biogasverwertungseinrichtung zu beeinträchtigen.

Es ist dabei besonders vorteilhaft, wenn während des An- oder Abfahrens eines Fermenters Schwachgas dem Fermenter über den Spülgaseinlass zugeführt wird, da es dann in diesen Phasen ermöglicht wird, Gutgas in diesem Fermenter zu gewinnen und zu entnehmen.

Vorteilhafterweise weist die Biogasanlage eine Abluft-/ Abgasleitung auf, die mit dem Gasauslass des mindestens einen Fermenters verbindbar ist und zu einer Abluftbehandlung führt. Dies ermöglicht es, Grenzwerte so unabhängig voneinander vorzugeben, dass Gasgemische, die in Fermentern entstehen und aufgrund ihrer Zusammensetzung weder dem Gutnoch dem Schwachgas zugeordnet werden können, sicher entsorgt werden können.

Vorteilhafterweise wird dabei mindestens einer der Gasspeicher oberhalb des mindestens einen Fermenters angeordnet, so dass er großflächig an diesen angrenzt. Dabei werden vorzugsweise mindestens 50% der Fläche des mindestens einen Fermenters bedeckt. Besonders vorzugsweise ist die gesamte Fläche des mindestens einen Fermenters oder besonders vorteilhaft aller vorhandenen Fermenter von dem/den Gasspeicher(n) bedeckt. Durch diese Bauweise lässt sich in der überwiegenden Zeit des Jahres eine besonders günstige Wärmedämmung des Fermenters erreichen, wobei gleichzeitig durch die flächige Ausführung des/der Gasspeicher(s) diese eine große Oberfläche zur Umgebung aufweisen, was im Jahresmittel eine gute Gaskühlung ermöglicht, ohne dass über die Grundfläche der Fermenter hinaus gehende Aufstellfläche benötigt wird.

### KURZBESCHREIBUNG DER ZEICHNUNGEN

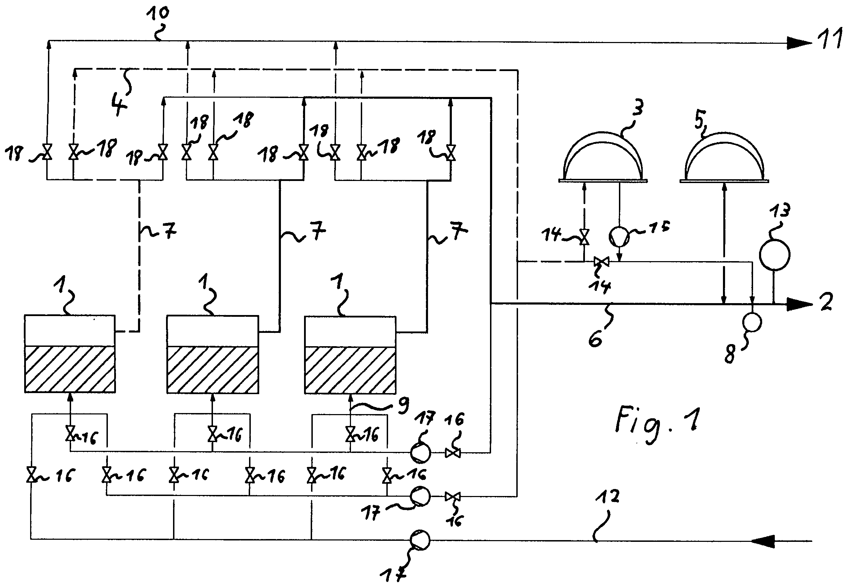

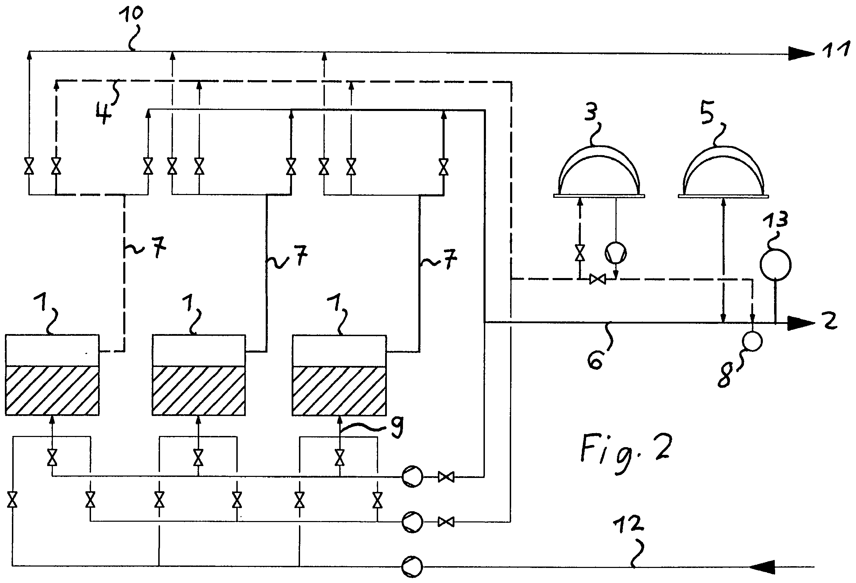

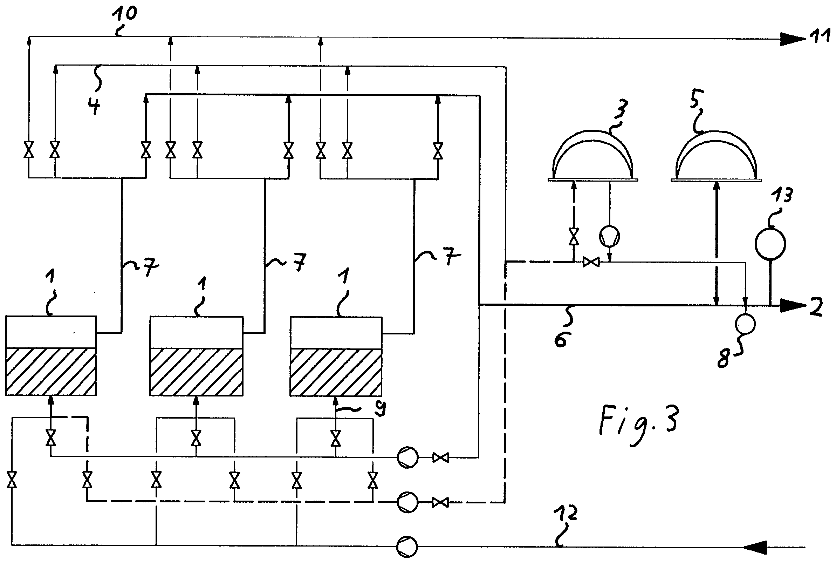

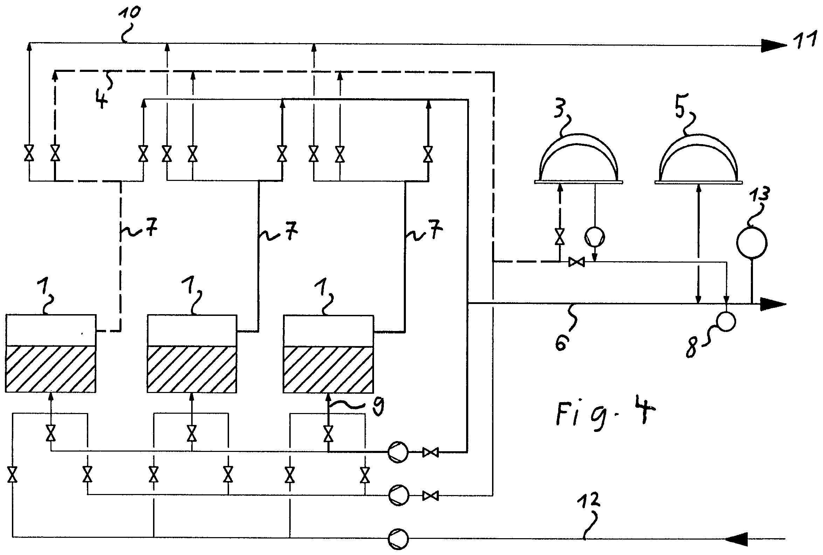

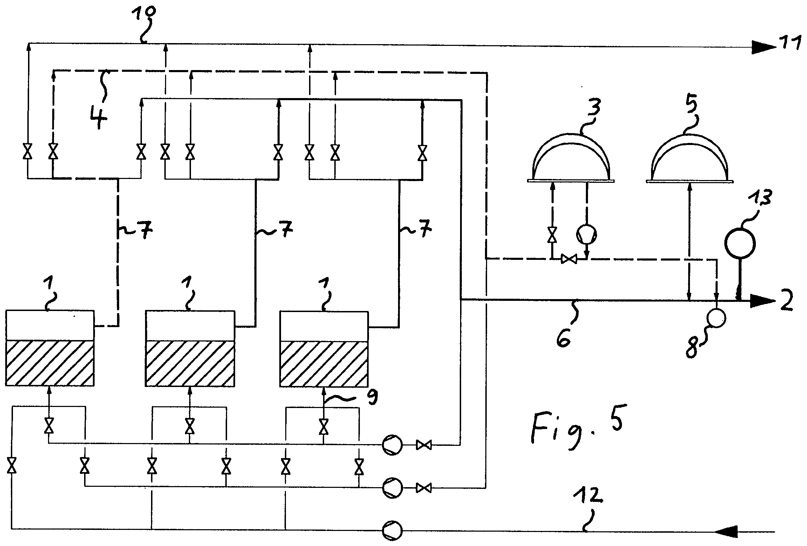

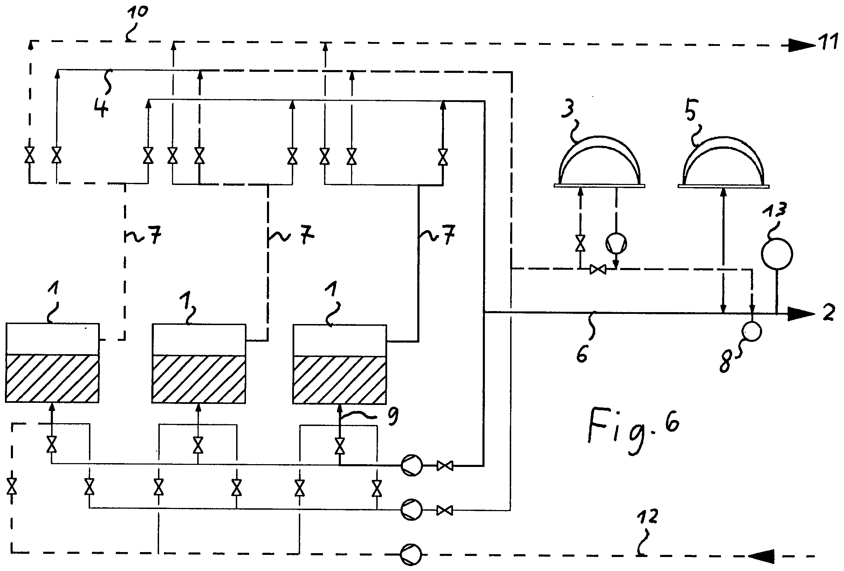

Die Erfindung wird im Folgenden anhand der Figuren 1 bis 10 schematisch näher erläutert. Es zeigen:
- Figur 1: - ein schematisches Verfahrensfließbild einer beispielhaften erfindungsgemäßen Biogasanlage während der Produktion von Gutgas in zwei Fermentern bei gleichzeitiger Speicherung von Schwachgas aus einem dritten Fermenter,
- Figur 2: - das Fließbild der Anlage aus Figur 1 während Schwachgas dem Gutgas vor dessen Verwertung zugemischt wird,
- Figur 3: - das Fließbild der Anlage aus den Figuren 1 und 2 während der Zufuhr von Schwachgas aus dem Schwachgasspeicher in einen Fermenter bei gleichzeitiger Entnahme von Gutgas aus sämtlichen Fermentern,
- Figur 4: - das Fließbild der in den Figuren 1 bis 3 dargestellten Anlage während der Produktion und Verwertung von Gutgas in zwei Fermentern bei gleichzeitiger Speicherung von Schwachgas aus einem Fermenter, wobei einem der Gutgas produzierenden Fermenter ein Teil des Gutgasstroms wieder zugeführt wird,
- Figur 5: - ein Fließbild der in den Figuren 1 bis 4 dargestellten Anlage während der Produktion von Gutgas in zwei Fermentern und der Produktion von Schwachgas in einem Fermenter, wobei Gutgas und Schwachgas als Mischgas verwertet werden und der Schwachgasanteil im Mischgas durch Entnahme und/oder Zufuhr das Schwachgases zum oder vom Schwachgasspeicher geregelt werden,
- Figur 6: - ein Fließbild der Anlage aus den Figuren 1 bis 5, wobei ein Fermenter Schwachgas erzeugt, welches gemeinsam mit dem Gutgas aus einem anderen Fermenter verwertet wird, wobei der Anteil des Schwachgases über den Schwachgasspeicher geregelt wird, wobei ein Teil des Gutgases in den Gutgas produzierenden Fermenter zurück geführt wird und ein dritter Fermenter gleichzeitig mit Frischluft gespült wird, die über ein Abluft-/ Abgasleitung abgeführt wird,
- Figuren 7 bis 10: - zeigen unterschiedliche beispielhafte Aufstellungsmöglichkeiten mit der Platzierung der Gasspeicher auf den Dächern der Fermenter oder neben den Fermentern.

### BESTER WEG ZUR AUSFÜHRUNG DER ERFINDUNG

Die beispielhafte erfindungsgemäße Anlage weist drei unabhängig voneinander betreibbare Fermenter 1 auf, welche mit ihren Gasauslässen 7 wahlweise mit der ersten Biogasleitung 4, der zweiten Biogasleitung 6 oder einer Abluft-/Abgasleitung 10 verbunden werden können, wobei letztere zu einer Abluft-/Abgasbehandlung 11 führt. Die in den Biogasleitungen 4 und 6 geführten Biogasströme können miteinander gemischt oder einzeln einer Biogasverwertungseinrichtung 2 zugeführt werden. Die beispielhafte erfindungsgemäße Anlage weist dafür zur Mischung der beiden Biogasströme eine Steuereinrichtung 8 auf. Weiterhin ist eine Messeinrichtung 13 vorgesehen, die den Methangehalt des der Verwertungseinrichtung 2 zugeführten Biogases messen kann. Die Biogasleitung 4 ist mit dem als Schwachgasspeicher ausgelegten Biogasspeicher 3 verbunden. Dabei kann durch Absperrarmaturen 14 sowie eine Fördereinrichtung 15 gezielt Biogas dem ersten Biogasspeicher 3 zugeführt oder diesem entnommen werden. Alternativ ist es möglich, diesen Speicher zu überbrücken.

Die zweite Biogasleitung 6 ist mit einem zweiten Biogasspeicher 5 verbunden, der als Ausgleichsbehälter für diese Biogasleitung ausgelegt ist.

Die Fermenter 1 weisen weiterhin Gaseinlässe 9 auf, welche durch Absperrarmaturen 16 wahlweise und unabhängig voneinander mit der Biogasleitung 4, der zweiten Biogasleitung 6 oder einer Frischluftzuleitung 12 verbunden werden können.

Dabei sind Fördereinrichtungen 17 vorgesehen, die dazu dienen, bei der Spülung der Fermenter 1 über den Gaseinlass 9, der sich im Bereich des Bodens des jeweiligen Fermenters befindet, einen ausreichenden Druck das Kühlgases bereit zu stellen. Weitere Absperrarmaturen 18 dienen dazu, die Gasauslässe 7 der Fermenter 1 selektiv und unabhängig voneinander mit der Abluft-/Abgasleitung 10 der ersten Biogasleitung 4 und/oder der zweiten Biogasleitung 6 zu verbinden.

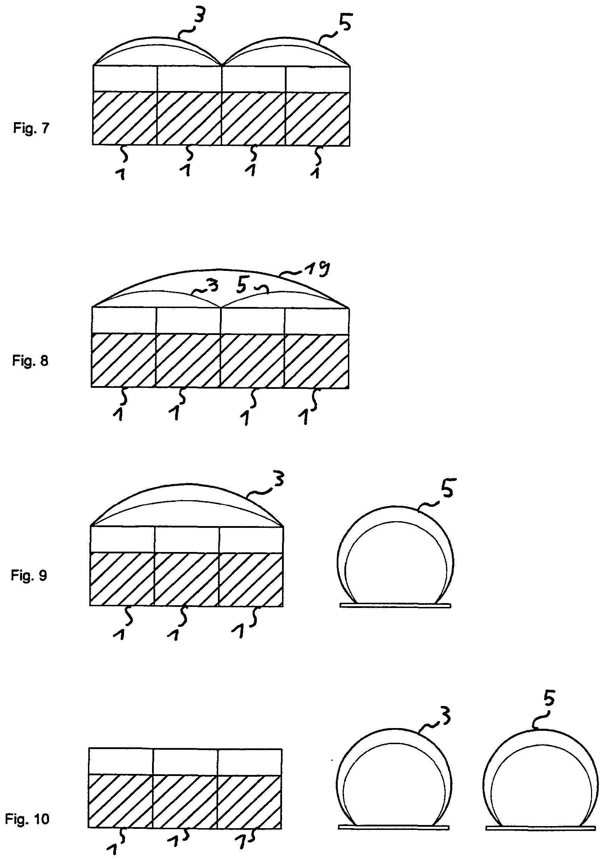

Die Biogasspeicher 3 und 5 können in konventioneller Weise unabhängig von den Fermentern 1 aufgestellt werden. Es ist jedoch auch möglich und besonders vorteilhaft, die Biogasspeicher so auszuführen, dass entweder einer der beiden Biogasspeicher, d.h. entweder der erste Biogasspeicher 3 oder der zweite Biogasspeicher 5, die Dachfläche der Fermenter bedeckt. Ebenfalls ist es möglich, beide Biogasspeicher auf einer von den vorhandenen Fermentern 1 gebildeten gemeinsamen Dachfläche anzuordnen. Dabei können, wie in Figur 7 dargestellt, voneinander unabhängige Biogasspeicher nebeneinander auf dem Dach angeordnet werden. Es ist jedoch auch möglich, wie in Figur 8 dargestellt, die beiden Biogasspeicher 3 und 5 mit einer gemeinsamen Schutzhülle 19 zu versehen.

## Patentansprüche

1. Biogasanlage, mindestens einen Fermenter (1), vorzugsweise einen Trockenfermenter zur Erzeugung von Biogas, eine Biogasverwertungseinrichtung (2), einen ersten Gasspeicher (3) zur Speicherung von Biogas mit einem Methangehalt unterhalb eines ersten vorgebbaren Grenzwertes, der mit einer ersten mit einem Gasauslass (7) des mindestens einen Fermenters (1) verbundenen Biogasleitung (4) verbunden ist, und einen zweiten Gasspeicher (5) zur Speicherung von Biogas mit einem Methangehalt oberhalb eines zweiten vorgebbaren Grenzwertes, der mit einer zweiten mit einem Gasauslass (7) des mindestens einen Fermenters (1) verbundenen Biogasleitung (6) verbunden ist, aufweisend, wobei die Biogasverwertungseinrichtung (2) mit dem ersten Biogasspeicher (3) und/oder der ersten Biogasleitung (4) und dem zweiten Biogasspeicher (5) und/ oder der zweiten Biogasleitung (6) verbunden ist, wobei die Biogasanlage eine Steuereinrichtung (8) zur Steuerung der Mengenverhältnisse der aus den Biogasleitungen (4, 6) und/oder den Biogasspeichern (3, 5) zur Biogasverwertungseinrichtung geführten Biogasströme abhängig von deren Methangehalt aufweist, wobei die Biogasanlage eine Messeinrichtung zur Messung des Methangehalts aufweist, die mit den Biogasleitungen (4, 6) und/oder den Biogasspeichern (3, 5) verbunden ist.

2. Biogasanlage nach Anspruch 1,
**dadurch gekennzeichnet,**
**dass** der mindestens eine Fermenter (1) einen mit einer Frischluftzuleitung (12) verbindbaren Spülgaseinlass (9), vorzugsweise im Bereich des Bodens des mindestens einen Fermenters (1), aufweist.

3. Biogasanlage nach Anspruch 1 oder 2,
**dadurch gekennzeichnet,**
**dass** der mindestens eine Fermenter (1) einen mit dem ersten Biogasspeicher (3) und/oder der ersten Biogasleitung (4) verbindbaren Spülgaseinlass (9), vorzugsweise im Bereich des Bodens des mindestens einen Fermenters, aufweist.

4. Biogasanlage nach einem der Ansprüche 1 bis 3,
**dadurch gekennzeichnet,**
**dass** der mindestens eine Fermenter (1) einen mit dem zweiten Biogasspeicher (5) und/oder der zweiten Biogasleitung (6) verbindbaren Spülgaseinlass (9), vorzugsweise im Bereich des Bodens des mindestens einen Fermenters (1), aufweist.

5. Biogasanlage nach einem der Ansprüche 1 bis 4,
**dadurch gekennzeichnet,**
**dass** mindestens einer der Gasspeicher (3, 5) oberhalb des mindestens einen Fermenters (1) angeordnet ist und großflächig an diesen angrenzt, wobei vorzugsweise mindestens 50% der Fläche des mindestens einen Fermenters (1), besonders vorzugsweise die gesamte Fläche des mindestens einen Fermenters (1), vorteilhafterweise aller vorhandener Fermenter (1) von dem/den Gasspeicher(n) (3, 5), bedeckt ist.

6. Biogasanlage nach einem der Ansprüche 1 bis 5,
**dadurch gekennzeichnet,**
**dass** die Biogasanlage eine Abluft-/Abgasleitung (10) aufweist, die mit dem Gasauslass (7) des mindestens einen Fermenters (1) verbunden ist und vorzugsweise zu einer Abluft-/Abgasbehandlung (11) führt.

7. Verfahren zum Betrieb einer Biogasanlage nach einem der Ansprüche 1 bis 6, wobei in einem Fermenter (1) gewonnenes Biogas, welches einen Methangehalt unterhalb eines ersten vorgebbaren Grenzwertes aufweist, in einem ersten Gasspeicher (3) zwischengespeichert wird.

8. Verfahren nach Anspruch 7,
**dadurch gekennzeichnet,**
**dass** das Biogas, welches einen Methangehalt unterhalb eines vorgebbaren Grenzwertes aufweist, mit einem in Fermentern (1) gewonnenen Biogas, welches einen Methangehalt oberhalb eines zweiten vorgebbaren Grenzwertes aufweist, gemischt und eine Biogasverwertungseinrichtung (2) mit einer Mischung der beiden Biogase betrieben wird.

9. Verfahren nach einem der Ansprüche 7 oder 8,
**dadurch gekennzeichnet,**
**dass** das Biogas, welches einen Methangehalt oberhalb eines zweiten vorgebbaren Grenzwertes aufweist, in einem zweiten Gasspeicher (5) zwischengespeichert wird.

10. Verfahren nach einem der Ansprüche 7 bis 9,
**dadurch gekennzeichnet,**
**dass** die Mengenverhältnisse der Biogase, insbesondere abhängig vom Methangehalt der Biogase, geregelt werden, vorzugsweise so, dass sich der Methangehalt der Mischung der beiden Biogase oberhalb eines dritten vorgebbaren Grenzwertes bewegt.

11. Verfahren nach einem der Ansprüche 7 bis 10,
**dadurch gekennzeichnet,**
**dass** während des An- oder Abfahrens eines Fermenters (1) Biogas, welches einen Methangehalt unterhalb des ersten vorgebbaren Grenzwertes aufweist, diesem Fermenter (1) zugeführt wird, wobei gleichzeitig Biogas, welches einen Methangehalt oberhalb des zweiten vorgebbaren Grenzwertes aufweist, diesem Fermenter (1) entnommen wird.

12. Verfahren nach einem der Ansprüche 7 bis 11,
**dadurch gekennzeichnet,**
**dass**, zumindest phasenweise und/oder zyklisch, Biogas, welches einen Methangehalt oberhalb des zweiten vorgebbaren Grenzwertes aufweist, einem Fermenter (1) zugeführt wird, wobei gleichzeitig Biogas, welches einen Methangehalt oberhalb des zweiten vorgebbaren Grenzwertes aufweist, und/oder Biogas, welches einen Methangehalt unterhalb des ersten vorgebbaren Grenzwertes aufweist, diesem Fermenter (1) entnommen wird.

13. Verfahren nach einem der Ansprüche 7 bis 12,
**dadurch gekennzeichnet,**
**dass** der erste Grenzwert so gewählt wird, dass der Methangehalt im ersten Biogasspeicher (3) unterhalb der vom Sauerstoffgehalt abhängigen unteren Explosionsgrenze liegt.

14. Verfahren nach einem der Ansprüche 7 bis 13,
**dadurch gekennzeichnet,**
**dass** der zweite Grenzwert bei 37% Methangehalt, vorzugsweise bei 52% Methangehalt liegt.

15. Verfahren nach einem der Ansprüche 7 bis 14,
**dadurch gekennzeichnet,**
**dass** der dritte Grenzwert bei 35% Methangehalt, vorzugsweise bei 50% Methangehalt liegt.

## Claims

1. Biogas plant, comprising at least one fermenter (1), preferably a dry fermenter for generating biogas, a biogas recovery device (2), a first gas reservoir (3) for storing biogas having a methane content below a first predeterminable threshold limit, which is connected to a first biogas pipe (4) which is connected to a gas outlet (7) of the least one fermenter (1), and a second gas reservoir (5) for storing biogas having a methane content above a second predeterminable threshold limit, which is connected to a second biogas pipe (6) connected to a gas outlet (7) of the at least one fermenter (1), wherein the biogas recovery device (2) is connected to the first biogas reservoir (3) and/or to the first biogas pipe (4) and to the second biogas reservoir (5) and/or to the second biogas pipe (6), wherein the biogas plant has a control device (8) for controlling the volume ratios of the biogas streams guided from the biogas pipes (4, 6) and/or from the biogas reservoirs (3, 5) to the biogas recovery unit dependent on their methane content, wherein the biogas plant has a measuring unit for measuring the methane content, which (measuring unit) is connected to the biogas pipes (4, 6) and/or to the biogas reservoirs (3, 5) .

2. Biogas plant according to claim 1,
**characterised in that**
the at least one fermenter (1) has a flushing gas inlet (9) which can be connected to a fresh air supply line (12), preferably in the region of the bottom of the at least one fermenter (1).

3. Biogas plant according to claim 1 or 2,
**characterised in that**
the at least one fermenter (1) has a flushing gas inlet (9) which can be connected to the first biogas reservoir (3) and/or to the first biogas pipe (4), preferably in the region of the bottom of the at least one fermenter.

4. Biogas plant according to one of clams 1 to 3,
**characterised in that**
the at least one fermenter (1) has a flushing gas inlet (9) which can be connected to the second biogas reservoir (5) and/or to the second biogas pipe (6), preferably in the region of the bottom of the at least one fermenter (1).

5. Biogas plant according to one of clams 1 to 4,
**characterised in that**
at least one of the gas reservoirs (3, 5) is arranged above the at least one fermenter (1) and adjoins same over a large surface area wherein preferably at least 50% of the surface of the at least one fermenter (1), and more preferably the entire surface of the at least one fermenter (1), and more advantageously of all of the fermenters (1) provided, is covered by the gas reservoir (s) (3, 5).

6. Biogas plant according to one of claims 1 to 5,
**characterised in that**
the biogas plant has an exhaust air/exhaust gas pipe (10) which is connected to the gas outlet (7) of the at least one fermenter (1) and preferably leads to an exhaust air/exhaust gas treatment area (11).

7. Method for operating a biogas plant according to one of claims 1 to 6,
wherein biogas obtained in a fermenter (1) and which has a methane content below a first predeterminable threshold limit, is temporarily stored in a first gas reservoir (3) .

8. Method according to claim 7
**characterised in that**
the biogas, which has a methane content below a predeterminable threshold limit, is mixed with a biogas which is obtained in the fermenters (1) and which has a methane content above a second predeterminable threshold limit, and a biogas recovery device (2) is operated with a mixture of the two biogases.

9. Method according to one of claims 7 or 8,
**characterised in that**
the biogas which has a methane content above a second predeterminable threshold limit, is temporarily stored in a second gas reservoir (5).

10. Method according to one of claims 7 to 9,
**characterised in that**
the volume ratios of biogases are regulated, in particular dependent on the methane content of the biogases, preferably so that the methane content of the mixture of the two biogases moves above a third predeterminable threshold limit.

11. Method according to one of claims 7 to 10,
**characterised in that**
during starting up and shutting down a fermenter (1) biogas which has a methane content below the first predeterminable threshold limit is supplied to this fermenter (1) wherein at the same time biogas which has a methane content above the second predeterminable threshold limit, is removed from this fermenter (1).

12. Method according to one of claims 7 to 11,
**characterised in that**
biogas which has a methane content above the second predeterminable threshold limit is supplied at least in phases and/or cyclically to a fermenter (1), wherein at the same time biogas which has a methane content above the second predeterminable threshold limit, and/ or biogas, which has a methane content below the first predeterminable threshold limit, is/are removed from this fermenter (1).

13. Method according to one of claims 7 to 12,
**characterised in that**
the first threshold limit is selected so that the methane content in the first biogas reservoir (3) lies below the lower explosion limit dependent on the oxygen content.

14. Method according to one of claims 7 to 13,
**characterised in that**
the second threshold limit lies at 37% methane content, preferably at 52% methane content.

15. Method according to one of claims 7 to 14,
**characterised in that**
the third threshold limit lies at 35% methane content, preferably at 50% methane content.

## Revendications

1. Installation de biogaz comprenant au moins un fermenteur (1), de préférence un fermenteur à sec, pour la production d'un biogaz, une installation de valorisation de biogaz (2), un premier réservoir de gaz (3) pour le stockage de biogaz ayant une teneur en méthane inférieure à une première valeur limite pouvant être prédéterminée, lequel est relié à une première conduite de biogaz (4) reliée à une sortie de gaz (7) de l'au moins un fermenteur (1), et un deuxième réservoir de biogaz (5) pour le stockage de biogaz ayant une teneur en méthane supérieure à une deuxième valeur limite pouvant être prédéterminée, lequel est relié à une deuxième conduite de biogaz (6) reliée à une sortie de gaz (7) de l'au moins un fermenteur (1), sachant que l'installation de valorisation de biogaz (2) est reliée au premier réservoir de biogaz (3) et / ou à la première conduite de biogaz (4) et au deuxième réservoir de biogaz (5) et /ou à la deuxième conduite de biogaz (6), l'installation de biogaz présentant un dispositif de commande (8) pour la commande des rapports quantitatifs des flux de biogaz en ce qui concerne leur teneur en méthane, lesquels sont conduits des conduites de biogaz (4, 6) et/ ou des réservoirs de biogaz (3, 5) à l'installation de valorisation de biogaz (2), sachant que l'installation de valorisation de biogaz présente un dispositif de mesure qui, destiné à mesurer la teneur en méthane, est relié aux conduites de biogaz (4, 6) et/ou aux réservoirs de biogaz (3, 5).

2. Installation de biogaz selon la revendication 1,
**caractérisée en ce que**,
de préférence dans la zone du fond de l'au moins un fermenteur (1), l'au moins un fermenteur (1) présente une entrée de gaz de purge (9), qui peut être reliée à une conduite d'alimentation en air frais (12).

3. Installation de biogaz selon revendication 1 ou 2,
**caractérisée en ce que**
l'au moins un fermenteur (1) présente, de préférence dans la zone du fond de l'au moins un fermenteur, une entrée de gaz de purge (9), qui peut être reliée au premier réservoir de biogaz (3) et / ou à la première conduite de biogaz (4).

4. Installation de biogaz selon l'une des revendications 1 à 3,
**caractérisée en ce que**
l'au moins un fermenteur (1) présente, de préférence dans la zone du fond de l'au moins un fermenteur (1), une entrée de gaz de purge (9), qui peut être reliée au deuxième réservoir de biogaz (5) et / ou à la deuxième conduite de biogaz (6).

5. Installation de biogaz selon l'une des revendications 1 à 4,
**caractérisée en ce que**
l'un des réservoirs de biogaz (3, 5) au moins est disposé au-dessus de l'au moins un fermenteur (1) et est largement adjacent à celui-ci, sachant que, préférentiellement au moins 50% de la surface de l'au moins un fermenteur (1), en particulier, de préférence, la surface totale de l'au moins un fermenteur (1) est couverte par le / les réservoirs de biogaz (3, 5).

6. Installation de biogaz selon l'une des revendications 1 à 5,
**caractérisée en ce que**
l'installation de biogaz présente une conduite d'air d'échappement / gaz d'échappement (10) qui est reliée à la sortie de gaz (7) de l'au moins un fermenteur (1) et qui, de préférence, conduit à un dispositif de traitement d'air d'échappement / gaz d'échappement (11).

7. Procédé d'exploitation d'une installation de biogaz selon l'une des revendications 1 à 6, sachant que le gaz obtenu dans un fermenteur (1), lequel présente une teneur en méthane située au-dessous d'une première valeur limite pouvant être prédéterminée, est temporairement stocké dans un premier réservoir de biogaz (3).

8. Procédé selon la revendication 7,
**caractérisé en ce que**
le biogaz qui présente une teneur en méthane inférieure à une valeur limite pouvant être prédéterminée, est mélangé avec un biogaz obtenu dans des fermenteurs (1) qui présente une teneur en méthane supérieure à une deuxième valeur limite pouvant être prédéterminée, et qu'une installation de valorisation de biogaz (2) est exploitée avec un mélange des deux biogaz.

9. Procédé selon l'une des revendications 7 ou 8,
**caractérisé en ce que**
le biogaz qui présente une teneur en méthane supérieure à une deuxième valeur limite pouvant être prédéterminée est temporairement stocké dans un deuxième réservoir de biogaz (5).

10. Procédé selon l'une des revendications 7 à 9,
**caractérisé en ce que**
les rapports quantitatifs des biogaz, en particulier en ce qui concerne la teneur en méthane des biogaz, sont régulés de sorte que la teneur en méthane du mélange des deux biogaz soit située au-dessus d'une troisième valeur limite pouvant être prédéterminée.

11. Procédé selon l'une des revendications 7 à 10,
**caractérisé en ce que**,
lors du démarrage ou de l'arrêt d'un fermenteur (1), du biogaz présentant une teneur en méthane inférieure à la première valeur limite pouvant être prédéterminée est conduit à ce fermenteur (1), sachant que du biogaz présentant une teneur en méthane supérieure à la deuxième valeur limite pouvant être prédéterminée est retiré simultanément de ce fermenteur (1).

12. Procédé selon l'une des revendications 7 à 11,
**caractérisé en ce que**,
au moins par phase et / ou cycle, du biogaz présentant une teneur en méthane supérieure à la deuxième valeur limite pouvant être prédéterminée est conduit à un fermenteur (1), sachant que du biogaz présentant une teneur en méthane supérieure à la deuxième valeur limite pouvant être prédéterminée et / ou du biogaz présentant une teneur en méthane inférieure à la première valeur limite pouvant être prédéterminée est retiré simultanément de ce fermenteur (1).

13. Procédé selon l'une des revendications 7 à 12,
**caractérisé en ce que**
la première valeur limite est choisie de sorte que la teneur en méthane dans le premier réservoir de biogaz (3) soit située au-dessous de la limite inférieure d'explosibilité, en ce qui concerne la teneur en oxygène.

14. Procédé selon l'une des revendications 7 à 13,
**caractérisé en ce que**
la deuxième valeur limite est de 37 % de méthane, de préférence de 52 % de méthane.

15. Procédé selon l'une des revendications 7 à 14,
**caractérisé en ce que**
la troisième valeur limite est de 35 % de méthane, de préférence de 50 % de méthane.
